# EUROPEAN PATENT SPECIFICATION

(11) **EP 4 209 245 B1**
(45) Date of publication and mention of the grant of the patent: **19.11.2025**
(21) Application number: 21864158.7
(22) Date of filing: 23.08.2021
(51) Int. Cl.: A61M 25/09, A61B 5/0215

(54) **GUIDEWIRE AND BIOLOGICAL SENSOR MANUFACTURING METHOD**
FÜHRUNGSDRAHT UND HERSTELLUNGSVERFAHREN FÜR BIOLOGISCHEN SENSOR
FIL-GUIDE ET PROCÉDÉ DE FABRICATION DE CAPTEUR BIOLOGIQUE

(30) Priority: 02.09.2020 JP 2020147429
(43) Date of publication of application: 12.07.2023
(73) Proprietor: Nipro Corporation, Osaka-shi, Osaka 531-8510 (JP)
(72) Inventor: NARUSE, Natsumi, Osaka-shi, Osaka 531-8510 (JP); UEHARA, Yohei, Osaka-shi, Osaka 531-8510 (JP); YAMAMOTO, Hirohito, Osaka-shi, Osaka 531-8510 (JP)
(74) Representative: Kraus & Lederer PartGmbB
(86) International application number: PCT/JP2021/030741
(87) International publication number: WO 2022/050100

(56) References cited:
- WO-A1-2012/173697
- JP-A- 2010 106 340
- JP-A- 2010 106 340
- JP-A- 2014 158 099
- JP-A- 2014 158 099
- JP-A- 2014 530 639
- JP-A- 2014 530 639
- JP-A- 2018 038 792
- JP-A- 2018 038 792

## Description

### TECHNICAL FIELD

The present invention relates to a guide wire inserted into a lumen of a living body and used to measure a pressure of fluid in the lumen, and a method for manufacturing a living body sensor used in the guide wire.

### BACKGROUND ART

As a method for measuring a pressure of fluid in a lumen of a living body, for example, a blood pressure in a coronary artery, a method in which a guide wire having a pressure sensor is inserted into a blood vessel is known. Patent Documents 1 and 2 each disclose a pressure measurement device in which a sensor for pressure detection is disposed in a housing provided at a tip end portion of a guide wire.

The above-described sensor includes a diaphragm and a resistive body provided to the diaphragm. While the guide wire is inserted into the blood vessel, a blood pressure is applied to the diaphragm of the sensor. When the diaphragm is bowed by the blood pressure, an electrical resistance value of each resistive body is changed. With this, a current flowing through the resistive body is changed. Furthermore, in a case in which a plurality of resistive bodies is provided, a potential difference is generated between each of the resistive bodies. The blood pressure is calculated based on a change in the current or the potential difference.

### PRIOR ART DOCUMENT

### PATENT DOCUMENT

Patent Document 1: Published Japanese Translation of PCT International Application No. 2010-540114
Patent Document 2: Japanese Patent Application Laid-Open No. 2018-38792

### SUMMARY OF THE INVENTION

At the time of measurement, a portion of the sensor comes into contact with blood. Therefore, it is desirable that the sensor (especially, conductive portion of the sensor) be coated for waterproof insulation. The conductive portion is a terminal provided at a portion where a conductive wire for connecting the sensor to a calculation device is connected to the sensor, for example.

However, in a case of the pressure measurement device disclosed in Patent Document 2, following problems occur. In the pressure measurement device, both the diaphragm and the terminal are located on a surface perpendicularly intersecting with an axial line direction of the guide wire. Thus, if coating is applied on the surface, the coating is applied not only on the terminal but also on the diaphragm, and the diaphragm becomes hard to be bowed.

Thus, it is conceivable to apply the coating only on the terminal without applying the coating on the diaphragm. However, the surface on which the diaphragm and the terminal are located is an extremely small surface having a diameter smaller than that of the lumen of the living body. Especially, by locating the diaphragm on the surface perpendicularly intersecting with the axial line direction of the guide wire, an advantage is obtained that even when the diaphragm approaches a blood vessel wall, pressing load to the blood vessel wall is hard to affect a deformation of the diaphragm, however, on the contrary, it is difficult to apply the coating with masking only a part (diaphragm portion) of such a surface. Even if the masking can be performed, since an accuracy of the masking becomes low, a part of the coating may be put on the diaphragm.

On the other hand, when a thin coating is applied to the entire of the surface on which the diaphragm and the terminal are located, the diaphragm becomes hard to move, and an accuracy of the pressure detection becomes low.

The present invention has been made in view of the above-described circumstances, and an object thereof is to provide a guide wire in which a conductive portion is coated with an insulating film without impairing mobility of a diaphragm, the guide wire having a configuration in which both the diaphragm and the conductive portion are on a surface intersecting with an axial line direction of the guide wire.

### MEANS FOR SOLVING THE PROBLEMS

(1) A wire for pressure measurement according to the present invention includes a guide wire main body having a distal end portion having an internal space, and a sensor located in the internal space of the distal end portion. The sensor includes a sensor main body having a surface intersecting with an axial direction of the guide wire main body and facing a distal end, a diaphragm located on the surface, a resistive body having a variable electrical resistance value by being deformed together with the diaphragm, a terminal connected to the resistive body, a conductive wire electrically connected to the terminal, and an insulating film formed by electrodeposition coating on a portion of the terminal and the conductive wire, the portion exposed in the surface, without electrodeposition coating on the diaphragm.
   According to the above-described configuration, the diaphragm is not coated with the insulating film, and the terminal and the conductive wire which are conductive portions are coated with the insulating film. Therefore, fluid can be prevented from touching the conductive portions without impairing mobility of the diaphragm.
(2) The sensor main body has a through hole opening in the surface and extending in the axial direction. The terminal is located at least at the opening or the through hole. The conductive wire is inserted into the through hole.
   The opposite side of the through hole with respect to the surface of the conductive wire can be easily covered with an insulator made of resin or the like, while coating a connection portion of the conductive wire to the terminal with the insulating film.
(3) The sensor main body has a columnar shape, and a diameter of the surface is smaller than 0.5 mm.
   Although it is difficult to coat only on the conductive portion of the surface because the diameter of the surface is smaller than 0.5 mm, according to the above-described configuration, since the insulating film is formed by electrodeposition coating, it is possible to coat only on the conductive portion on a small surface and to prevent coating on the diaphragm.
(4) Preferably, the insulating film contains polyimide as a main component.
(5) The present invention is directed to a method for manufacturing a living body sensor located in an internal space of a distal end portion of a guide wire main body having the distal end portion having the internal space, the living body sensor obtained by insulation coating on a terminal and a conductive wire exposed in a surface facing a distal end of the sensor, without insulation coating on a diaphragm exposed in the surface. The method for manufacturing the living body sensor according to the present invention includes an electrodeposition step of applying a potential to the terminal through the conductive wire in a state where at least the surface of the sensor is immersed in a coating liquid, a water washing step of washing away a coating component contained in the coating liquid attached at least to the diaphragm in the sensor, and a baking step of heating the coating component attached to the sensor after washing with water.

According to the above-described manufacturing method, since the insulating film is coated on the surface by electrodeposition coating, only the terminal and the conductive wire which are conductive portions can be coated with the insulating film in the surface. In other words, the diaphragm is not coated with the insulating film. Therefore, fluid can be prevented from touching the conductive portion without impairing mobility of the diaphragm.

Furthermore, the potential is applied to the terminal through the conductive wire in the electrodeposition step. In other words, time and effort for using an electric wire other than the conductive wire when applying the potential in the electrodeposition step and attaching the conductive wire after the electrodeposition step are not necessary. Additionally, completion of a formation of the insulating film can be grasped by monitoring a current in the electrodeposition step.

### EFFECTS OF THE INVENTION

According to the present invention, in a configuration in which both the diaphragm and the conductive portion are on the surface intersecting with the axial line direction of the guide wire, the conductive portion can be coated with the insulating film, without impairing mobility of the diaphragm.

### BRIEF DESCRIPTION OF THE DRAWINGS

Fig. 1 is a schematic view of a guide wire 10 according to an embodiment of the present invention.
Fig. 2 is an enlarged cross-sectional view along a cutting line II-II in Fig. 1.
Fig. 3 is an enlarged cross-sectional view along a cutting line III-III in Fig. 1.
Fig. 4 is a perspective view of a pressure sensor 11.
Fig. 5 is a cross-sectional view along a cutting line V-V in Fig. 4.
Fig. 6 is a view seen from an arrow VI in Fig. 4.
Fig. 7 is a circuit diagram of a bridge circuit according to the embodiment of the present invention.
Fig. 8 is a schematic view showing an electrodeposition step.
Fig. 9 is a schematic view showing a baking step.

### MODES FOR CARRYING OUT THE INVENTION

Hereinafter, a preferred embodiment of the present invention is described. Note that it is needless to say that the present embodiment is merely one embodiment of the present invention, and that the embodiment can be changed without departing from the gist of the present invention.

### [Guide wire 10]

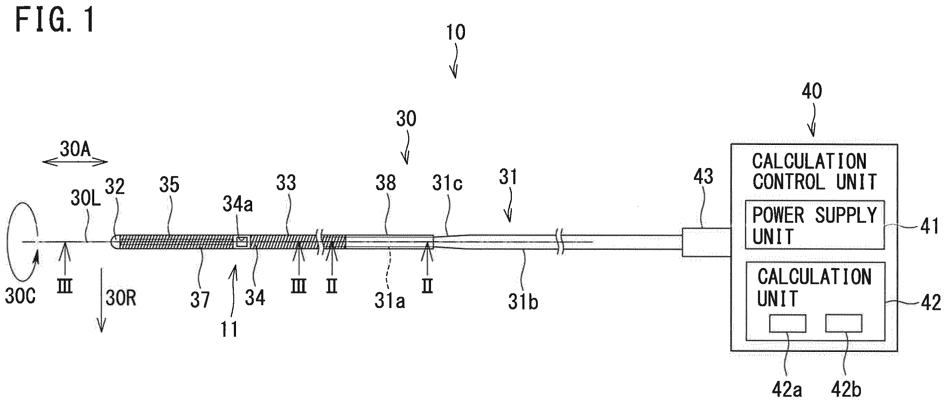

As shown in Fig. 1, a guide wire 10 according to the present embodiment includes a guide wire main body 30 and a pressure sensor 11 (an example of a sensor and a living body sensor) provided to the guide wire main body 30. A calculation control unit 40 is electrically connected to one end of the guide wire main body 30. In Fig. 1, of both ends of the guide wire main body 30, a fixed end (end connected to the calculation control unit 40) is a proximal end (right side in Fig. 1), and a free end (tip end when inserted into a blood vessel) is a distal end (left side in Fig. 1). Hereinafter, in the guide wire main body 30, a side where there is the proximal end is referred to as a proximal side, and a side where there is the distal end is referred to as a distal side.

The guide wire main body 30 is an elongated rope body and is insertable into the blood vessel such as a coronary artery. The pressure sensor 11 is provided at an end portion on the distal side of the guide wire main body 30. The calculation control unit 40 calculates a blood pressure based on electrical information (voltage value) output from the pressure sensor 11. In other words, the guide wire 10 is used to measure the blood pressure.

In Figs. 1 to 3, an axial center line 30L of the guide wire main body 30 is shown. In the present specification, directions relating to components constituting the guide wire main body 30, that is, an axial direction 30A, a radial direction 30R, and a circumferential direction 30C are defined as follows. The axial direction 30A, the radial direction 30R, and the circumferential direction 30C are defined based on the axial center line 30L in a straight state in which the guide wire main body 30 is not bowed and curved, in other words, based on the straight axial center line 30L. The axial direction 30A is a direction parallel to the axial center line 30L, and is a direction including both a distal direction and a proximal direction. The radial direction 30R is all directions perpendicularly intersecting with the axial center line 30L. The circumferential direction 30C is a direction around the axial center line 30L.

### [Guide wire main body 30]

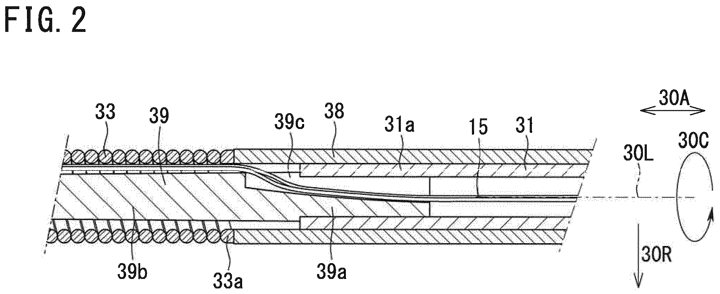

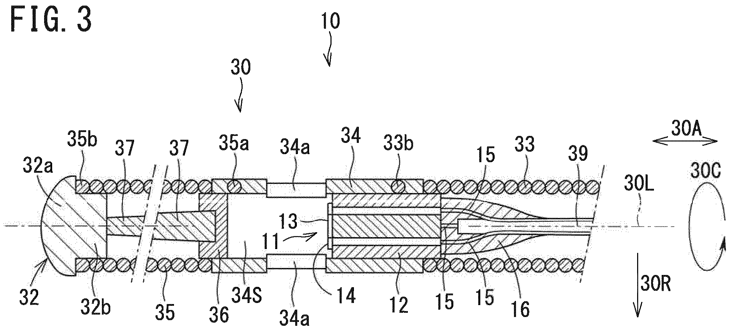

As shown in Fig. 1, the guide wire main body 30 includes a core wire 31, a tip end guide portion 32, a first helical body 33, a housing 34, a second helical body 35, and a guide tube 38. As shown in Fig. 2, the guide wire main body 30 includes a taper pin 39. As shown in Fig. 3, the guide wire main body 30 includes a coupling wall 36 and a tip end wire 37.

As shown in Fig. 1, the core wire 31 is a member constituting a framework of the guide wire main body 30. The core wire 31 provides a certain mechanical strength to bending of the guide wire main body 30 so that the guide wire main body 30 can be inserted into the blood vessel without being bent. The core wire 31 is a cylindrical wire material and extends from the proximal end to the distal side. The material of the core wire 31 is a medical stainless steel, for example. The axial center line of the core wire 31 is parallel to the axial center line 30L.

The core wire 31 is easy to be bowed in the distal side than in the proximal side. The core wire 31 has a small diameter portion 31a located on the distal side, a large diameter portion 31b located on the proximal side, and a tapered portion 31c coupling the small diameter portion 31a to the large diameter portion 31b. The small diameter portion 31a and the large diameter portion 31b each have a constant outer diameter, and the outer diameter of the large diameter portion 31b is larger than the outer diameter of the small diameter portion 31a. The outer diameter of the tapered portion 31c is equal to the outer diameter of the large diameter portion 31b at the proximal end, gradually decreases from the proximal end toward the distal end, and is equal to the outer diameter of the small diameter portion 31a at the distal end. Since the outer diameter of the core wire 31 gradually decreases toward the distal side, rigidity of the core wire 31 decreases in the order of the large diameter portion 31b, the tapered portion 31c, and the small diameter portion 31a.

As shown in Fig. 2, the taper pin 39 is disposed on the distal side of a distal end portion of the core wire 31. As with the core wire 31, the taper pin 39 is also a member constituting the framework of the guide wire main body 30, and provides a certain mechanical strength to bending of the guide wire main body 30.

The taper pin 39 includes a shaft portion 39a located on the proximal side and a tapered portion 39b extending from the shaft portion 39a to the distal side. The outer diameter of the shaft portion 39a is constant. The shaft portion 39a is inserted into the small diameter portion 31a of the core wire 31. The shaft portion 39a is fixed to the small diameter portion 31a by laser welding or an adhesive, for example. The outer diameter of the tapered portion 39b is formed so as to be tapered toward the distal side. Thus, rigidity of the tapered portion 39b gradually decreases toward the distal side. Since a distal end portion of the guide wire main body 30 at which the taper pin 39 is disposed is easy to be bent, the guide wire main body 30 is easy to be guided along the blood vessel. Furthermore, a groove 39c opening in an outer circumferential surface of the taper pin 39 is formed from a proximal end of the taper pin 39 to a proximal side portion of the tapered portion 39b in parallel to the axial direction 30A. Four conductive wires 15 (to be described later) of the pressure sensor 11 pass through the core wire 31 via the groove 39c and are connected to the calculation control unit 40.

As shown in Figs. 1 and 2, the guide tube 38 is located outside the small diameter portion 31a of the core wire 31 in the radial direction 30R, and covers a distal side portion of the small diameter portion 31a. The shape of the guide tube 38 is a cylindrical shape. The axial center line of the guide tube 38 is parallel to the axial center line 30L. The guide tube 38 is fixed to an outer peripheral surface of the small diameter portion 31a of the core wire 31. The guide tube 38 has flexibility. The guide tube 38 is made of a medical synthetic resin, for example, and is thermally welded to an outer peripheral surface of the core wire 31, for example.

As shown in Figs. 1 and 3, the tip end guide portion 32 is disposed at the distal end of the guide wire main body 30. The tip end guide portion 32 is a member guiding a traveling direction of the guide wire main body 30 so as to follow the blood vessel by abutting on a blood vessel wall when the guide wire main body 30 is inserted into the blood vessel. The tip end guide portion 32 includes a hemispherical portion 32a located on the distal side and a columnar portion 32b extending from the hemispherical portion 32a to the proximal side. The hemispherical portion 32a has a hemispherical shape protruding to the distal side so as not to damage the blood vessel wall. The outer diameter of the hemispherical portion 32a is substantially equal to the outer diameter of the second helical body 35. The columnar portion 32b protrudes from the hemispherical portion 32a to the proximal side, and has a columnar shape having an outer diameter smaller than the outer diameter of the hemispherical portion 32a. By inserting the columnar portion 32b into the second helical body 35, the position of the tip end guide portion 32 is determined with respect to the second helical body 35, and the outer surfaces of the hemispherical portion 32a and the second helical body 35 continue smoothly without a step. The material of the tip end guide portion 32 is a medical stainless steel, for example.

As shown in Figs. 1 and 3, the first helical body 33 and the second helical body 35 are provided on the distal side of the guide wire main body 30. The first helical body 33 and the second helical body 35 have bending rigidity smaller than that of the taper pin 39, that is, are easy to be bent. The first helical body 33 is formed of a wire material wound in a helical shape. The material of the first helical body 33 is a medical stainless steel, for example. The axial center line of the first helical body 33 is parallel to the axial center line 30L. As shown in Figs. 2 and 3, the tapered portion 39b of the taper pin 39 is inserted into the first helical body 33. The first helical body 33 has a proximal end portion 33a (see Fig. 2) and a distal end portion 33b (see Fig. 3). As shown in Fig. 2, the proximal end portion 33a is fixed to an outer peripheral surface of the tapered portion 39b of the taper pin 39 by laser welding or an adhesive, for example. With this, bending rigidity of the first helical body 33 is reinforced by the taper pin 39.

As shown in Fig. 1, the housing 34 (an example of a distal end portion) is provided on the distal side of the guide wire main body 30. As shown in Figs. 1 and 3, the housing 34 has a cylindrical shape and has an internal space 34S. The pressure sensor 11 is accommodated in the internal space 34S of the housing 34. The material of the housing 34 is a medical stainless steel, for example. The axial center line of the housing 34 is parallel to the axial center line 30L. To a proximal end portion of the housing 34, the distal end portion 33b of the first helical body 33 is fixed by laser welding or an adhesive, for example.

The housing 34 has a plurality of through holes 34a. In the present embodiment, the housing 34 has two through holes 34a. The through hole 34a penetrates a cylindrical-shaped wall of the housing 34 along the radial direction 30R. The internal space 34S of the housing 34 and the outside communicate via the through holes 34a. The two through holes 34a are disposed along the circumferential direction 30C of the guide wire main body 30 at intervals of 180 degrees around the axial center line 30L. Note that the number of the through holes 34a is not limited to two. The intervals between the through holes 34a is not limited to 180 degrees around the axial center line 30L.

As shown in Figs. 1 and 3, the second helical body 35 is formed of a wire material wound in a helical shape. The material of the second helical body 35 is a medical stainless steel, for example. The axial center line of the second helical body 35 is parallel to the axial center line 30L. As shown in Fig. 3, the second helical body 35 has a proximal end portion 35a and a distal end portion 35b. The proximal end portion 35a of the second helical body 35 is fixed to a distal end portion of the housing 34. The second helical body 35 and the housing 34 are fixed by laser welding or an adhesive, for example. The columnar portion 32b of the tip end guide portion 32 is inserted into the distal end portion 35b of the second helical body 35. The distal end portion 35b is fixed to an outer peripheral surface of the columnar portion 32b. The second helical body 35 and the tip end guide portion 32 are fixed by laser welding or an adhesive, for example.

The coupling wall 36 is a member for coupling the tip end wire 37 to the housing 34. The coupling wall 36 is fixed to the distal end portion of the housing 34. The coupling wall 36 is made of a metal solder material, for example.

The tip end wire 37 reinforces bending rigidity of the second helical body 35. The tip end wire 37 is a wire material made of a medical stainless steel, for example. The axial center line of the tip end wire 37 is parallel to the axial center line 30L. A proximal end portion of the tip end wire 37 is fixed to the coupling wall 36. A distal end portion of the tip end wire 37 is fixed to the columnar portion 32b of the tip end guide portion 32 by laser welding or an adhesive, for example.

By the above-described configuration, the taper pin 39 and the tip end guide portion 32 are coupled via the first helical body 33, the housing 34, and the second helical body 35. Furthermore, the housing 34 and the tip end guide portion 32 are coupled via the tip end wire 37. The taper pin 39 is fixed to the core wire 31. In this manner, the guide wire main body 30 itself (excluding the core wire 31) is supported by the core wire 31 and mechanical strength is provided.

By such a configuration, when an operation of delivering the guide wire main body 30 to the blood vessel is performed at the proximal end, the guide wire main body 30 advances in the blood vessel without being bent, following this operation. Furthermore, when the tip end guide portion 32 comes into contact with the blood vessel wall, the guide wire main body 30 is curved along the blood vessel wall.

### [Pressure sensor 11]

As shown in Fig. 3, the pressure sensor 11 is disposed in the internal space 34S of the housing 34. A proximal side portion of the internal space 34S is almost filled with the pressure sensor 11. On the other hand, a distal side portion of the internal space 34S, in other words, the internal space 34S located on the distal side of the pressure sensor 11 exists as a space. The through hole 34a of the housing 34 is opened in the distal side portion of the internal space 34S.

As shown in Figs. 3 to 6, the pressure sensor 11 includes a sensor main body 12, a diaphragm 13, a bridge circuit 14, the four conductive wires 15, a covering member 16, and insulating films 23.

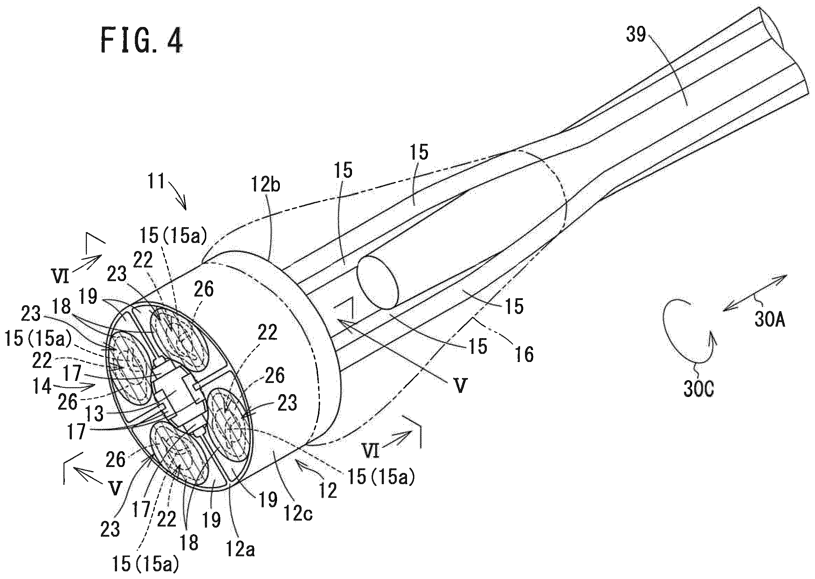

As shown in Fig. 4, the shape of the sensor main body 12 is a columnar shape. The axial center line of the sensor main body 12 is parallel to the axial center line 30L. The sensor main body 12 has a distal end surface 12a (an example of a surface), a proximal end surface 12b, and an outer circumferential surface 12c. The distal end surface 12a and the proximal end surface 12b are surfaces perpendicularly intersecting with the axial direction 30A. The distal end surface 12a faces the distal end. The proximal end surface 12b faces the proximal end. The outer circumferential surface 12c faces the radial direction 30R. The material of the sensor main body 12 is monocrystalline silicon, for example.

In the present embodiment, the diameter of the sensor main body 12 (diameter of a circle of the columnar shape) is approximately 0.3 mm. In other words, the diameters of the distal end surface 12a and the proximal end surface 12b are approximately 0.3 mm or smaller. Note that the diameter of the sensor main body 12 (diameters of the distal end surface 12a and the proximal end surface 12b) is not limited to approximately 0.3 mm. Although it is preferable that the diameter of the sensor main body 12 be smaller than 0.5 mm, it may be 0.5 mm or larger.

The diaphragm 13, the bridge circuit 14, and the four conductive wires 15 are attached to the sensor main body 12.

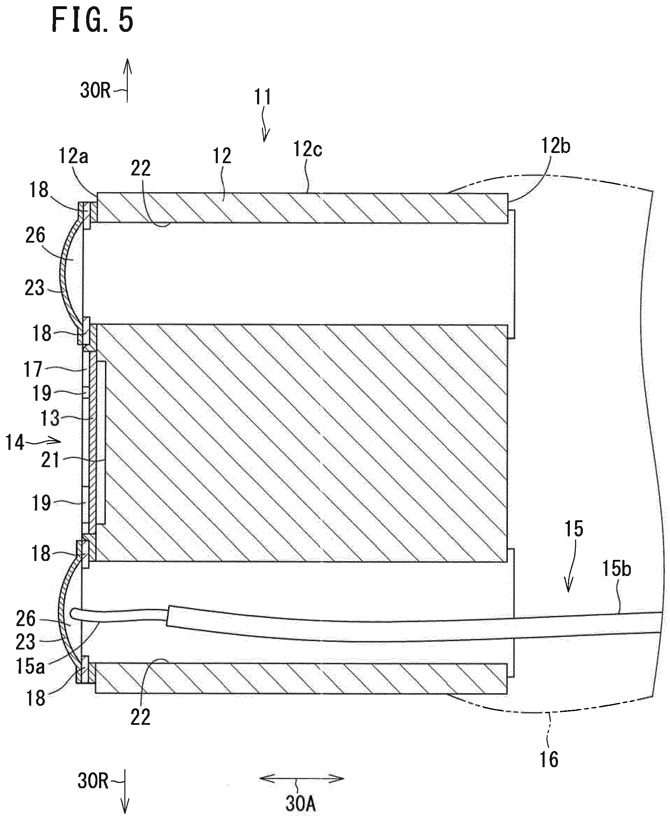

As shown in Fig. 5, the sensor main body 12 has a recessed portion 21 in the distal end surface 12a. The recessed portion 21 is for facilitating a deformation of the diaphragm 13 by a pressure of fluid in a lumen. When viewed from the distal side of the sensor main body 12, the shape of the recessed portion 21 is a circle. The depth of the recessed portion 21 in the axial direction 30A is constant. The axial center line of the recessed portion 21 coincides with the axial center line of the sensor main body 12.

As shown in Figs. 4 to 6, the sensor main body 12 has four through holes 22. The four through holes 22 are disposed along the circumferential direction 30C at intervals of 90 degrees around the axial center line of the sensor main body 12. Each through hole 22 extends along the axial direction 30A and is open to both the distal end surface 12a and the proximal end surface 12b of the sensor main body 12. When viewed from the axial direction 30A, the shape of the through hole 22 is a circle.

As shown in Figs. 4 to 6, the diaphragm 13 is disposed on the distal end surface 12a of the sensor main body 12 and is fixed thereto. The material of the diaphragm 13 is monocrystalline silicon, for example, and the diaphragm 13 is formed by partially etching the sensor main body 12. The shape of the diaphragm 13 is a circular shape when viewed from the axial direction 30A, and is a rectangular shape when viewed from the radial direction 30R. The axial center line of the diaphragm 13 coincides with the axial center line of the sensor main body 12. The distal end surface 12a, the diaphragm 13, and the recessed portion 21 are disposed coaxially. The outer diameter of the diaphragm 13 is larger than the diameter of an inner peripheral surface of the recessed portion 21. The diaphragm 13 covers the entire opening of the recessed portion 21. A portion of the diaphragm 13, the portion covering the opening of the recessed portion 21, is elastically deformable when an external force is applied.

As shown in Figs. 4, 6, and 7, the bridge circuit 14 is disposed on the distal end surface 12a of the sensor main body 12 and is fixed thereto. The bridge circuit 14 includes four resistive bodies 17 (17A, 17B), four terminals 18 (18A, 18B, 18C, 18D), and four connecting bodies 19. The bridge circuit 14 surrounds the diaphragm 13.

The bridge circuit 14 is a full-bridge circuit in which the four resistive bodies 17 all function as strain gauges for measurement. Thus, the four resistive bodies 17 are comprised of two types of resistive bodies having different resistance change characteristics. The two types of resistive bodies are a first resistive body 17A and a second resistive body 17B. In the present specification, when it is not necessary to distinguish between the first resistive body 17A and the second resistive body 17B, these are referred to as resistive bodies 17.

The four resistive bodies 17 are fixed on a surface on the distal side of the diaphragm 13. The material of the resistive bodies 17 is boron, for example. When viewed from the axial direction 30A, the four resistive bodies 17 are fixed to an outer peripheral portion of the diaphragm 13. The four resistive bodies 17 are disposed along the circumferential direction 30C at intervals of 90 degrees around the axial center line of the sensor main body 12. Here, the first resistive bodies 17A and the second resistive bodies 17B are alternately disposed along the circumferential direction 30C. Note that the resistive bodies 17 are subjected to insulation treatment in advance.

Both the first resistive body 17A and the second resistive body 17B are semiconductors using a piezo resistance effect. Being fixed to the diaphragm 13, the resistive body 17 is elastically deformed with an elastic deformation of the diaphragm 13. When the resistive body 17 is elastically deformed, an electrical resistance value of the resistive body 17 is changed. In other words, the resistive body 17 has a variable electrical resistance value by being deformed together with the diaphragm 13.

The shapes of the first resistive body 17A and the second resistive body 17B are different from each other. The postures of the first resistive body 17A and the second resistive body 17B with respect to the diaphragm 13 are also different from each other. Such differences in the shape and the posture provides the above-described difference in the resistance change characteristics between the first resistive body 17A and the second resistive body 17B.

When viewed from the axial direction 30A, the shape of the first resistive body 17A is a Π shape. In the posture with respect to the diaphragm 13, the first resistive body 17A includes a circumferential direction component 51 and two radial direction components 52. The circumferential direction component 51 extends generally along a circumferential direction of the diaphragm 13. The radial direction components 52 extend generally along a radial direction of the diaphragm 13. The first resistive body 17A is configured so that the electrical resistance value thereof increases with the deformation of the diaphragm 13 at the time of pressurization.

When viewed from the axial direction 30A, the shape of the second resistive body 17B is a rectangular shape. In the posture with respect to the diaphragm 13, the second resistive body 17B is configured by a circumferential direction component extending generally along the circumferential direction of the diaphragm 13. The second resistive body 17B is configured so that the electrical resistance value thereof decreases with the deformation of the diaphragm 13 at the time of pressurization.

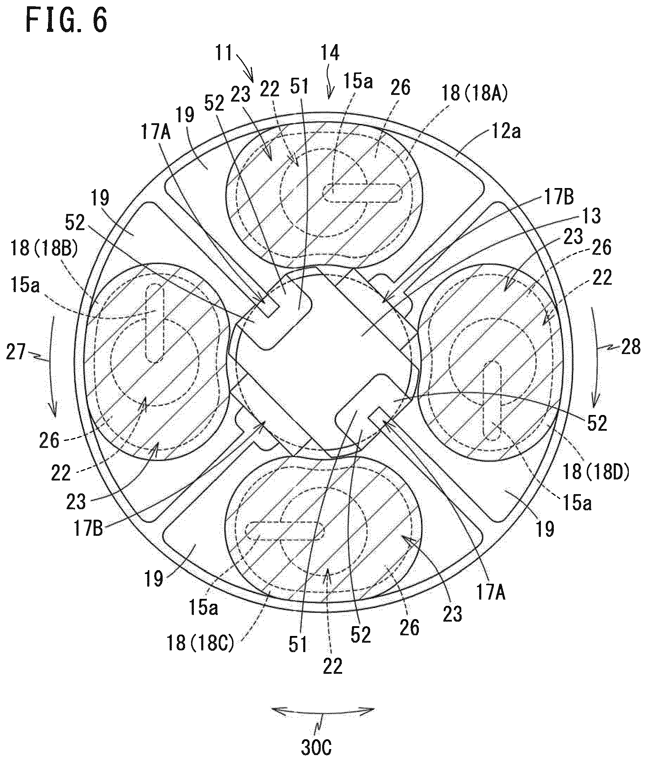

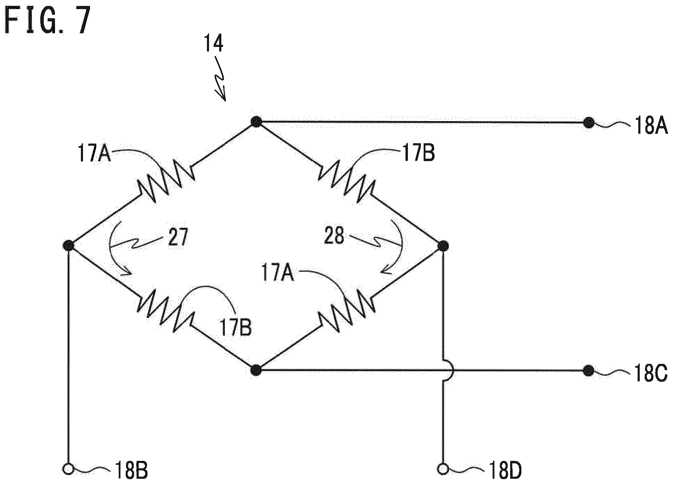

As shown in Figs. 6 and 7, the four terminals 18 are two input terminals 18A, 18C and two output terminals 18B, 18D in the bridge circuit 14. In the present specification, in a case in which it is not necessary to distinguish between the input terminals 18A, 18C and the two output terminals 18B, 18D, these are referred to as terminals 18. The material of the terminals 18 is a solder material, for example. As shown in Fig. 5, the four terminals 18 are four conductive layers respectively provided corresponding to the four through holes 22 of the sensor main body 12. The four terminals 18 have the conductive layers exposed to solder the conductive wires 15. The terminals 18 are each laminated on at least one of an inner surface of each through hole 22 or a periphery of an opening of each through hole 22 in the distal end surface 12a. In the present embodiment, the terminals 18 are each laminated around the periphery of the opening of each through hole 22 in the distal end surface 12a.

As shown in Figs. 4 and 6, the four terminals 18 are disposed outside the diaphragm 13 in the radial direction 30R. The four terminals 18 are disposed along the circumferential direction 30C at intervals of 90 degrees around the axial center line of the sensor main body 12. The four terminals 18 and the four resistive bodies 17 are alternately disposed in the circumferential direction 30C. Each terminal 18 is disposed between two adjacent resistive bodies 17 among the four resistive bodies 17.

As shown in Figs. 4 to 6, the four connecting bodies 19 are provided corresponding to the four terminals 18, respectively. Each connecting body 19 is a conductive layer laminated in the periphery of the opening of each through hole 22 in the distal end surface 12a. In the present embodiment, for each connecting body 19, an insulation processing is performed on its surface before the terminal 18 is electrically connected. And, with respect to the connecting body 19 on which the insulation processing is performed, the terminal 18 is in an electrically connected state to the connecting body 19. Each connecting body 19 electrically connects two adjacent resistive bodies 17 and a terminal 18 located between the two adjacent resistive bodies 17. In this manner, the four resistive bodies 17 and the four terminals 18 are alternately electrically connected.

As shown in Fig. 6, in the bridge circuit 14, the two input terminals 18A, 18C are disposed at intervals of 180 degrees from each other, and the two output terminals 18B, 18D are disposed at intervals of 180 degrees from each other. As shown in Figs. 6 and 7, the bridge circuit 14 includes two paths (one path 27 and other path 28) from one input terminal 18A toward the other input terminal 18C. The one path 27 is a path passing through the first resistive body 17A, one output terminal 18B, and the second resistive body 17B. The other path 28 is a path passing through the second resistive body 17B, the other output terminal 18D, and the first resistive body 17A. Here, the one input terminal 18A is on a high voltage side, and the other input terminal 18C is on a low voltage side.

In a state in which a voltage is applied between the two input terminals 18A, 18C, voltage drops occur in the order of the first resistive body 17A and the second resistive body 17B in the one path 27, and voltage drops occur in the order of the second resistive body 17B and the first resistive body 17A in the other path 28.

In a state in which the diaphragm 13 is not pressurized, the first resistive body 17A and the second resistive body 17B are not deformed. At this time, the electrical resistance values of the first resistive body 17A and the second resistive body 17B are the same. Therefore, no potential difference occurs between the two output terminals 18B, 18D.

On the other hand, in a state where the diaphragm 13 is pressurized, the first resistive body 17A and the second resistive body 17B are deformed. As described above, at the time of pressurizing, the electrical resistance value of the first resistive body 17A increases, and the electrical resistance value of the second resistive body 17B decreases. In other words, a voltage drop amount in the first resistive body 17A becomes larger than a voltage drop amount in the second resistive body 17B. Therefore, a potential difference occurs between the two output terminals 18B, 18D.

In a state in which the guide wire main body 30 is inserted into the blood vessel and a blood pressure is applied to the pressure sensor 11, a potential difference occurs between the two output terminals 18B, 18D in accordance with the blood pressure. The magnitude of the blood pressure can be identified based on the potential difference.

As shown in Fig. 5, the four conductive wires 15 are electrically connected to the four terminals 18, respectively. The conductive wire 15 has a conductive wire main body 15a formed of a conductor, and an insulating cover 15b formed of an insulator. The insulating cover 15b covers the conductive wire main body 15a except for both end portions of the conductive wire main body 15a. At a distal end portion of the conductive wire 15, the conductive wire main body 15a is electrically and mechanically connected to the terminal 18 by soldering. A solder 26 may be filled between the conductive wire main body 15a and the terminal 18, and may cover a part of the terminal 18. The solder 26 closes the openings of the distal end surface 12a of the four through holes 22 of the sensor main body 12. Note that a part of the solder 26 may enter the four through holes 22 of the sensor main body 12.

One end of the conductive wire 15 is electrically connected to the pressure sensor 11 by connecting the conductive wire main body 15a and the terminal 18. The conductive wire 15 extends from one end thereof and is inserted into the through hole 22. On the proximal side of the through hole 22, the conductive wire 15 passes through the core wire 31 via the groove 39c and is connected to the calculation control unit 40. In other words, one end of the conductive wire 15 is connected to the pressure sensor 11, and the other end of the conductive wire 15 is connected to the calculation control unit 40.

As shown in Figs. 3 to 5, the covering member 16 is provided on the proximal side of the sensor main body 12. In the present embodiment, the covering member 16 is made of an adhesive. The configuration of the covering member 16 is not limited to the adhesive, and may be an insulating material such as silicon, rubber, or resin. The covering member 16 is fixed to the proximal end surface 12b of the sensor main body 12 and protrudes from the proximal end surface 12b to the proximal side. The covering member 16 closes the openings of the proximal end surface 12b of the four through holes 22 of the sensor main body 12. In other words, the openings on both sides of the through hole 22 are closed by the solder 26 and the covering member 16, respectively. Note that a part of the covering member 16 may enter the four through holes 22 of the sensor main body 12.

As shown in Fig. 4, the covering member 16 is coupled to the taper pin 39 and is fixed to the taper pin 39. With this, the sensor main body 12 is fixed with respect to the taper pin 39.

### [Insulating film 23]

As shown in Fig. 5, the insulating films 23 are electrodeposition coated on a part of the distal end surface 12a. Specifically, the insulating films 23 are electrodeposition coated on a portion exposed in the distal end surface 12a of the terminals 18, the solder 26, and the conductive wires 15 disposed on the distal end surface 12a. In the present embodiment, the terminals 18 excluding peripheral edge portions thereof and the conductive wires 15 are covered with the solder 26, and the peripheral edge portions of the terminals 18 are not covered with the solder 26. In other words, of the terminals 18, the solder 26, and the conductive wires 15 disposed on the distal end surface 12a, the insulating film 23 is electrodeposition coated on the peripheral edge portions of the terminals 18 and the solder 26.

On the other hand, the insulating film 23 is not electrodeposition coated on the diaphragm 13 and the connecting bodies 19 disposed on the distal end surface 12a.

Note that the insulating films 23 are shown with a hatching in Figs. 4 and 6. As shown in the figures, four insulating films 23 exist independently from each other on the distal end surface 12a.

The insulating film 23 includes polyimide as a main component. Note that the insulating film 23 may include an insulator other than polyimide as the main ingredient, or may include epoxy or urethane as the main component, for example.

### [Calculation control unit 40]

As shown in Fig. 1, the calculation control unit 40 includes a power supply unit 41 that supplies current to the pressure sensor 11, a calculation unit 42 that performs calculation processing on electrical information output from the pressure sensor 11, and a connector 43 connected to the four conductive wires 15.

The power supply unit 41 is configured to apply a voltage to the bridge circuit 14 of the pressure sensor 11 through two conductive wires 15 connected to the two input terminals 18A, 18C (see Figs. 6 and 7).

The calculation unit 42 obtains a voltage value output from the bridge circuit 14 of the pressure sensor 11 through two conductive wires 15 connected to the two output terminals 18B, 18D (see Figs. 6 and 7). The calculation unit 42 calculates the blood pressure acting on the pressure sensor 11 based on a change in the obtained output voltage value. The calculation unit 42 includes a memory 42a. More specifically, the calculation unit 42 calculates the blood pressure in the following manner.

The memory 42a stores a correspondence relationship between the above-described output voltage value and the blood pressure as one-to-one correspondence data, for example. Therefore, when the output voltage value is obtained, the calculation unit 42 can identify the blood pressure corresponding to the output voltage value based on the correspondence relationship stored in the memory 42a. In this manner, the calculation unit 42 can calculate the blood pressure acting on the pressure sensor 11, based on the voltage value output from the pressure sensor 11.

### [Use example of guide wire 10]

The guide wire 10 is used to measure the blood pressure in a coronary artery, for example. The guide wire main body 30 is inserted into the coronary artery with the distal end provided with the tip end guide portion 32 as a head of an insertion direction into the blood vessel. The position of the guide wire main body 30 in the coronary artery is grasped based on the position of the tip end guide portion 32 projected on an X-ray fluoroscopic image of the blood vessel.

When the pressure sensor 11 reaches a measurement position of the blood pressure in the coronary artery, inserting of the guide wire main body 30 is interrupted. In such a state, a constant voltage is supplied from the power supply unit 41 to the pressure sensor 11 by a user's operation.

In the blood vessel, blood flows into the internal space 34S of the housing 34, and the blood pressure acts on the surface of the diaphragm 13 of the pressure sensor 11. With this, the diaphragm 13 is elastically deformed, and accordingly the electrical resistance values of the four resistive bodies 17 are changed.

In the blood flow, there occurs a pulsation in which rising and falling of the blood pressure are repeated by movements of a heart. The four resistive bodies 17 are elastically deformed following the pulsation of the blood flow. With this, the electrical resistance values of the four resistive bodies 17 are changed in accordance with the blood pressure of the pulsating blood flow.

The calculation unit 42 of the calculation control unit 40 obtains the electrical information output from the pressure sensor 11. As described above, the calculation unit 42 calculates the blood pressure acting on the pressure sensor 11 based on the electrical information.

### [Method for manufacturing pressure sensor 11]

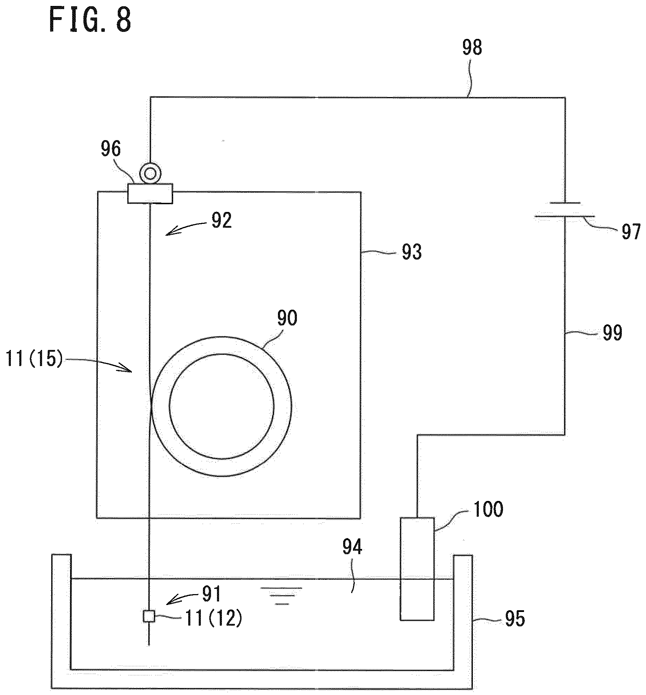

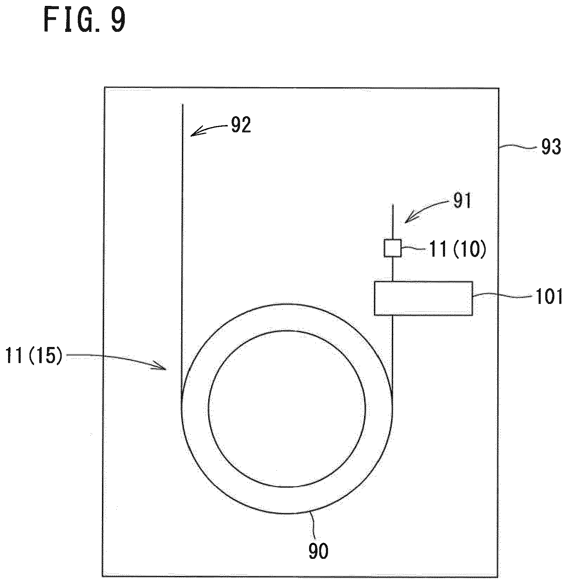

Hereinafter, a method for electrodeposition coating of the insulating film 23 in a manufacturing process of the pressure sensor 11 will be described with reference to Figs. 8 and 9.

As shown in Fig. 8, the conductive wire 15 of the pressure sensor 11 is wound around a spool 90. At this time, a distal end portion 91 (portion at which the sensor main body 12 is provided) of the conductive wire 15 and a proximal end portion 92 (portion connected to the connector 43) of the conductive wire 15 are not wound around the spool 90. The spool 90 is rotatably fixed or supported by a plate 93. Note that although the plate 93 is made of glass epoxy in the present embodiment, the material is not limited to the glass epoxy.

The distal end portion 91 is immersed in a coating liquid 94 stored in a container 95. In the present embodiment, the coating liquid 94 includes fine particles of polyimide. Note that the coating liquid 94 is selected appropriately in accordance with the type of the insulating film 23 to be electrodeposition coated. Furthermore, the coating liquid 94 may include substances other than polyimide.

The proximal end portion 92 is clipped to the plate 93 by a clip 96 made of metal. Furthermore, a conductive wire 98 connected to a cathode of a power supply 97 is clipped to the clip 96. With this, the proximal end portion 92 is electrically connected to the cathode of the power supply 97 via the clip 96 and the conductive wire 98. An electrode 100 is connected to an anode of the power supply 97 via a conductive wire 99. The electrode 100 is immersed in the coating liquid 94 stored in the container 95. In the present embodiment, the electrode 100 is made of stainless steel, but the material is not limited to stainless steel.

In the circuit configured as described above, the power supply 97 executes energization of a predetermined voltage (50 V in the present embodiment). With this, a potential of 50 V is applied to the terminals 18 and the solder 26 disposed on the distal end surface 12a of the sensor main body 12. In the present embodiment, the energization is performed for 20 seconds. Furthermore, in the present embodiment, the coating liquid 94 is stirred by a stirrer or the like during the energization. With this, fine particles of polyimide are diffused. By applying the potential, the fine particles of polyimide attach to the distal end portion 91 immersed in the coating liquid 94. The step in which the potential is applied corresponds to an electrodeposition step.

Next, the distal end portion 91 is taken out from the coating liquid 94 and is washed. In the present embodiment, washing is performed with pure water. By the washing, the fine particles of polyimide attached to the distal end surface 12a other than the conductive portion (the terminals 18 and the solder 26) are washed away. In other words, the fine particles of the polyimide attached to the diaphragm 13 and the connecting bodies 19 which are other than the conductive portion are washed away, whereas the fine particles of the polyimide remain attached to the terminals 18 and the solder 26. Note that since the connecting bodies 19 are covered with the insulator and the fine particles of polyimide do not directly attach thereto, the connecting bodies 19 are portions other than the conductive portion here. The step of washing corresponds to a water washing step.

Next, the sensor main body 12 of the distal end portion 91 is heated. In the present embodiment, as shown in Fig. 9, the distal end portion 91 is placed on the plate 93, and a weight 101 is put on near the distal end portion 91. In this state, the sensor main body 12 is heated. Heating of the sensor main body 12 is realized, for example, by blowing hot air toward the sensor main body 12 or by placing the sensor main body 12 on an oven or a hot plate. In a case in which the hot air is adopted as the heating method, by putting the weight 101 near the distal end portion 91, shaking of the sensor main body 12 due to blowing of the hot air can be prevented. The heating is performed in a range approximately from 170°C to 200°C for 30 minutes, for example. The fine particles of polyimide attached to the terminals 18 and the solder 26 are melted by the heating, and the insulating films 23 are formed by subsequent cooling. Note that the heating time, the temperature, and the heating measure for the pressure sensor 11 can be appropriately selected in addition to those described above. The step of heating corresponds to a baking step.

### [Actions and effects of the present embodiment]

According to the present embodiment, the diaphragm 13 is not coated with the insulating films 23, and the terminals 18 which are the conductive portions (when the conductive wires 15 and the solder 26 are exposed, the conductive wires 15 and the solder 26 in addition to the above-described one) are coated with the insulating films 23. Thus, fluid can be prevented from touching the conductive portion without impairing mobility of the diaphragm 13.

Furthermore, according to the present embodiment, the opposite side of the through hole 22 with respect to the distal end surface 12a in the conductive wire 15 can be covered with the covering member 16, while coating the connection portion to the terminal 18 in the conductive wire 15 with the insulating film 23.

Furthermore, when the diameter of the distal end surface 12a is smaller than 0.5 mm (0.28 mm in the present embodiment), it is difficult to coat only the conductive portion on the distal end surface 12a, however according to the present embodiment, since the insulating films 23 are electrodeposition coated, only the conductive portions can be coated in the small distal end surface 12a and coating on the diaphragm 13 can be prevented.

Furthermore, according to the present embodiment, the insulating films 23 can be made to have a thickness of approximately from 1 to 5 µm, for example. Furthermore, also in a portion having an uneven shape such as the terminals 18 or the solder 26, the insulating film 23 having a uniform thickness along the uneven shape can be formed. Furthermore, presence or absence of the insulating film 23 can be clearly grasped from the appearance.

Furthermore, according to the above-described method for manufacturing the pressure sensor 11, since the insulating films 23 are coated on the distal end surface 12a by electrodeposition coating, only the terminals 18, the solder 26, and the conductive wires 15 which are the conductive portions can be coated with the insulating films 23 on the distal end surface 12a. In other words, the diaphragm 13 is not coated with the insulating films 23. Thus, the fluid can be prevented from touching the conductive portion without impairing the mobility of the diaphragm 13. Furthermore, by adjusting the potential and time in the electrodeposition step, the thickness of the insulating film 23 can be controlled in units of 1 µm. With this, the fine particles of polyimide attached to the distal end portion 91 in the electrodeposition process can be prevented from sagging into the diaphragm 13. Furthermore, in the electrodeposition step, the insulation resistance value can be 1 MΩ or larger. With this, pinholes are hard to occur in the insulating film 23, and the thickness of the insulating film 23 is easy to be uniform.

Furthermore, according to the above-described method for manufacturing the pressure sensor 11, the potential is applied to the terminal 18 through the conductive wire 15 in the electrodeposition step. In other words, it is not necessary to use an electric wire other than the conductive wire 15 when the potential is applied in the electrodeposition step and to attach the conductive wire 15 after the electrodeposition step.

### [Modification examples]

Although an embodiment of the present invention is described in detail in the above, the above-described description is merely an example of the present invention in all respects. It is needless to say that various improvements or modifications can be made without departing from the scope of the present invention. With respect to each component of the guide wire 10 according to the above-described embodiment, the component may be omitted, replaced, or added appropriately in accordance with the form of embodiment. Furthermore, the shape and the size of each component of the guide wire 10 may be appropriately set in accordance with the form of embodiment. For example, following changes are possible.

In the above-described embodiment, the shape of the sensor main body 12 is a columnar shape, and the distal end surface 12a is perpendicular to the axial direction 30A of the guide wire main body 30. The sensor main body 12 only needs to have the distal end surface 12a facing the distal side, and the shape of the sensor main body 12 and the angle of the distal end surface 12a with respect to the axial direction 30A are not limited. The shape of the sensor main body 12 may be, for example, a prismatic shape, and the distal end surface 12a may be inclined with respect to the axial direction 30A.

In the above-described embodiment, the shape of the diaphragm 13 is a disc shape. The shape of the diaphragm 13 is not limited as long as the diaphragm 13 can be elastically deformed in accordance with a change in pressure applied to the diaphragm 13. The diaphragm 13 may be a plate-like member, and the shape of the plate-like member when viewed from the axial direction 30A may be arbitrary shape. The arbitrary shape is, for example, a polygonal shape, and includes a quadrangular shape, a hexagonal shape, an octagonal shape, and the like.

In the above-described embodiment, the four resistive bodies 17 are disposed at intervals of 90 degrees around the axial center line of the sensor main body 12. The disposition of the four resistive bodies 17 is not limited thereto. For example, the four resistive bodies 17 may be disposed around the axial center line of the sensor main body 12 at non-uniform intervals, for example, at intervals of 120 degrees, 60 degrees, 120 degrees and 60 degrees, or at intervals of 60 degrees, 90 degrees, 30 degrees, and 180 degrees.

In the above-described embodiment, the four through holes 22 for providing the four terminals 18 are disposed at intervals of 90 degrees around the axial center line of the sensor main body 12. The disposition of the four through holes 22 is not limited thereto. For example, as with the four resistive bodies 17, the four through holes 22 may be disposed around the axial center line of the sensor main body 12 at non-uniform intervals, for example, at intervals of 120 degrees, 60 degrees, 120 degrees and 60 degrees, or at intervals of 60 degrees, 90 degrees, 30 degrees, and 180 degrees. Furthermore, in the above-described embodiment, the shape of the through hole 22 when viewed from the axial direction 30A is a circle. The shape of the through hole 22 when viewed from the axial direction 30A may be, for example, a polygonal shape, and is not limited.

Although the four resistive bodies 17 and the four terminals 18 are provided in the above-described embodiment, the numbers of the resistive bodies 17 and the terminals 18 are not limited to four. The number of the conductive wires 15 is determined in accordance with the number of the resistive bodies 17 and the number of the terminals 18. When the number of the resistive bodies 17 and the number of the terminals 18 are other than four, the pressure sensor 11 has a circuit that is different from the bridge circuit 14 of the above-described embodiment and makes all the resistive bodies 17 function as strain gauges. Furthermore, the number of the insulating films 23 is not limited to four.

In the above-described embodiment, the surface of each connecting body 19 is subjected to insulation process before the terminal 18 is electrically connected. As with the terminals 18, the insulating films 23 may be coated by electrodeposition coating. Furthermore, the electrical connection between the terminal 18 and the conductive wire 15 is not limited to the solder 26. For example, instead of the solder 26, the terminal 18 and the conductive wire 15 may be electrically connected by a conductive adhesive, or a member in which the terminal 18 and the conductive wire 15 are integrally formed in an electrically connected state may be used.

### DESCRIPTION OF REFERENCE CHARACTERS

- 10: guide wire
- 11: pressure sensor (sensor, living body sensor)
- 12: sensor main body
- 12a: distal end surface (surface)
- 13: diaphragm
- 15: conductive wire
- 17: resistive body
- 18: terminal
- 22: through hole
- 23: insulating film
- 30: guide wire main body
- 30A: axial direction
- 34: housing (distal end portion)
- 34S: internal space

## Claims

1. A guide wire comprising:
a guide wire main body (30) having a distal end portion having an internal space; and
a sensor (11) located in the internal space of the distal end portion, wherein
the sensor (11) comprises:
a sensor main body (12) having a surface intersecting with an axial direction of the guide wire main body (30) and facing a distal end;
a diaphragm (13) located on the surface;
a resistive body (17) having a variable electrical resistance value by being deformed together with the diaphragm;
a terminal (18) connected to the resistive body(17);
a conductive wire (15) electrically connected to the terminal (18); and
an insulating film (23) formed by electrodeposition coating on a portion of the terminal (18) and the conductive wire (15), the portion exposed in the surface, without electrodeposition coating on the diaphragm.

2. The guide wire according to claim 1, wherein
the sensor main body (12) has a through hole opening in the surface and extending in the axial direction,
the terminal (18) is located at least at the opening or the through hole, and
the conductive wire (15) is inserted into the through hole.

3. The guide wire according to claim 1 or 2, wherein the sensor main body (12) has a columnar shape, and a diameter of the surface is smaller than 0.5 mm.

4. The guide wire according to any one of claims 1 to 3, wherein the insulating film (23) contains polyimide as a main component.

5. A method for manufacturing a living body sensor located in an internal space of a distal end portion of a guide wire main body (30) having the distal end portion having the internal space, the living body sensor obtained by insulation coating on a terminal and a conductive wire exposed in a surface facing a distal end of the sensor, without insulation coating on a diaphragm exposed in the surface, the method comprising,
an electrodeposition step of applying a potential to the terminal through the conductive wire in a state where at least the surface of the living body sensor is immersed in a coating liquid;
a water washing step of washing away a coating component contained in the coating liquid attached at least to the diaphragm in the living body sensor; and
a baking step of heating the coating component attached to the living body sensor after washing with water.

## Patentansprüche

1. Führungsdraht umfassend:
einen Führungsdrahthauptkörper (30) mit einem distalen Endabschnitt mit einem Innenraum; und
einen Sensor (11), der sich in dem Innenraum des distalen Endabschnitts befindet, wobei der Sensor (11) umfasst:
einen Sensorhauptkörper (12) mit einer Oberfläche, die sich mit einer axialen Richtung des Führungsdrahthauptkörpers (30) schneidet und einem distalen Ende zugewandt ist;
eine Membran (13), die sich auf der Oberfläche befindet;
einen Widerstandskörper (17), dessen elektrischer Widerstandswert sich durch Verformen zusammen mit der Membran ändert;
einen Anschluss (18), der mit dem Widerstandskörper (17) verbunden ist;
eine leitfähige Leitung (15), die elektrisch mit dem Anschluss (18) verbunden ist; und
einen Isolierfilm (23), der durch Elektroabscheidung auf einem Abschnitt des Anschlusses (18) und der leitfähigen Leitung (15) ausgebildet ist, wobei der Abschnitt bei der Oberfläche freiliegt, ohne dass Elektroabscheidung auf der Membran ausgebildet ist.

2. Führungsdraht nach Anspruch 1, wobei
der Sensorhauptkörper (12) ein Durchgangsloch aufweist, das sich in der Oberfläche öffnet und sich in der axialen Richtung erstreckt,
sich der Anschluss (18) zumindest an der Öffnung oder dem Durchgangsloch befindet, und
die leitfähige Leitung (15) in das Durchgangsloch eingeführt ist.

3. Führungsdraht nach Anspruch 1 oder 2, wobei der Sensorhauptkörper (12) eine säulenartige Form aufweist und ein Durchmesser der Oberfläche kleiner als 0,5 mm ist.

4. Führungsdraht nach einem der Ansprüche 1 bis 3, wobei der Isolierfilm (23) Polyimid als eine Hauptkomponente enthält.

5. Verfahren zum Herstellen eines Sensors für einen lebenden Körper, der sich in einem Innenraum eines distalen Endabschnitts eines Führungsdrahthauptkörpers (30) befindet, wobei der distale Endabschnitt den Innenraum aufweist, wobei der Sensor für einen lebenden Körper durch Isolierbeschichtung auf einem Anschluss und einer leitfähigen Leitung, ohne Isolierbeschichtung auf einer Membran, die bei der Oberfläche freiliegt, erhalten wird, wobei das Verfahren umfasst:
einen Elektroabscheidungsschritt eines Anlegens eines Potentials an den Anschluss durch die leitfähige Leitung in einem Zustand, während zumindest die Oberfläche des Sensors für einen lebenden Körper in eine Beschichtungsflüssigkeit eingetaucht wird;
einen Wasserwaschschritt, bei dem eine in der Beschichtungsflüssigkeit enthaltene Beschichtungskomponente, die zumindest an der Membran in dem Sensor angebracht ist, abgewaschen wird; und
einen Brennschritt, bei dem nach dem Waschen mit Wasser die an dem Sensor für einen lebenden Körper angebrachte Beschichtungskomponente erhitzt wird.

## Revendications

1. Fil-guide comprenant :
un corps principal de fil-guide (30) ayant une partie d'extrémité distale ayant un espace interne ; et
un capteur (11) situé dans l'espace interne de la partie d'extrémité distale, dans lequel
le capteur (11) comprend :
un corps principal de capteur (12) ayant une surface coupant une direction axiale du corps principal de fil-guide (30) et faisant face à une extrémité distale ;
un diaphragme (13) situé sur la surface ;
un corps résistif (17) ayant une valeur de résistance électrique variable en étant déformé conjointement avec le diaphragme ;
une borne (18) connectée au corps résistif (17) ;
un fil conducteur (15) connecté électriquement à la borne (18) ; et
un film isolant (23) formé par revêtement par électrodéposition sur une partie de la borne (18) et du fil conducteur (15), la partie étant exposée dans la surface, sans revêtement par électrodéposition sur le diaphragme.

2. Fil-guide selon la revendication 1, dans lequel
le corps principal de capteur (12) a un trou traversant s'ouvrant dans la surface et s'étendant dans la direction axiale,
la borne (18) est située au moins au niveau de l'ouverture ou du trou traversant, et
le fil conducteur (15) est inséré dans le trou traversant.

3. Fil-guide selon la revendication 1 ou 2, dans lequel le corps principal de capteur (12) a une forme colonnaire, et un diamètre de la surface est inférieur à 0,5 mm.

4. Fil-guide selon l'une quelconque des revendications 1 à 3, dans lequel le film isolant (23) contient du polyimide en tant que composant principal.

5. Procédé de fabrication d'un capteur de corps vivant situé dans un espace interne d'une partie d'extrémité distale d'un corps principal de fil-guide (30) ayant la partie d'extrémité distale ayant l'espace interne, le capteur de corps vivant étant obtenu par revêtement d'isolation sur une borne et un fil conducteur exposé dans une surface faisant face à une extrémité distale du capteur, sans revêtement d'isolation sur un diaphragme exposé dans la surface, le procédé comprenant,
une étape d'électrodéposition consistant à appliquer un potentiel à la borne à travers le fil conducteur dans un état où au moins la surface du capteur de corps vivant est immergée dans un liquide de revêtement ;
une étape de lavage à l'eau consistant à éliminer par lavage un composant de revêtement contenu dans le liquide de revêtement fixé au moins au diaphragme dans le capteur de corps vivant ; et
une étape de cuisson consistant à chauffer le composant de revêtement fixé au capteur de corps vivant après lavage à l'eau.
